# EUROPEAN PATENT APPLICATION

(11) **EP 2 275 410 A1**
(43) Date of publication of application: **19.01.2011**
(21) Application number: 09742741.3
(22) Date of filing: 07.05.2009
(51) Int. Cl.: C07D 213/75, A61K 31/44, A61P 43/00, C07C 311/07

(54) **PROCESS FOR PRODUCTION OF COMPOUND HAVING ANTAGONISTIC ACTIVITY ON NPYY5 RECEPTOR, AND USEFUL CRYSTAL**

(30) Priority: 08.05.2008 JP 2008122154
(71) Applicant: Shionogi&Co., Ltd., Osaka-shi Osaka 541-0045 (JP)
(72) Inventor: SUGATA, Yoshihide, Osaka-shi Osaka 553-0002 (JP); IDE, Yutaka, Osaka-shi Osaka 553-0002 (JP); SAKATA, Teruo, Amagasaki-shi Hyogo 660-0813 (JP); OMURA, Sohei, Amagasaki-shi Hyogo 660-0813 (JP)
(74) Representative: Vossius & Partner
(86) International application number: PCT/JP2009/058608
(87) International publication number: WO 2009/136617

(57) **Abstract**

Disclosed are process for producing a compound having NPYY5 receptor antagonism and useful crystals.
A process for producing a compound represented by the formula (IV): 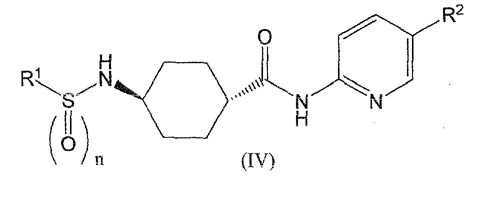 wherein R¹ is substituted or unsubstituted alkyl, R² is substituted or unsubstituted alkyl, and n is 1 or 2, or the like, which comprises reacting a compound represented by the formula (II): 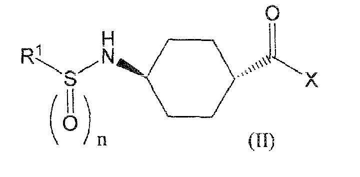 wherein R¹ and n are as defined, above, and X is a leaving group, or the like and a compound represented by formula (III): 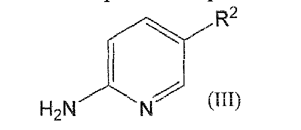 wherein R² is as defined above, or the like.

## Description

### [Field of the Invention]

The present invention relates to a process for producing a compound having NPYY5 receptor antagonism and to a useful crystal.

### [Background Art]

Patent Document 1 discloses a compound represented by the formula: 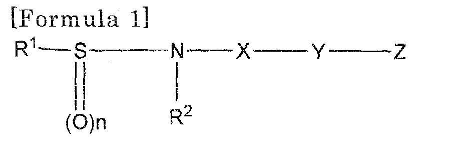 as a compound having NPYY5 receptor antagonism. As a general production process when Y is CONR⁷, the following scheme is described. 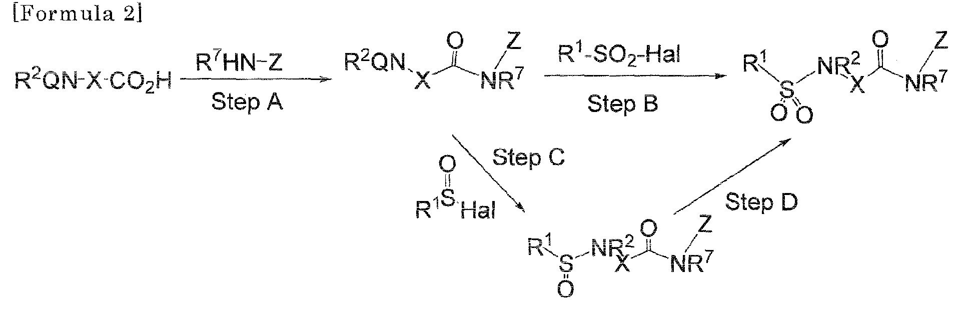 Patent Document 1 describes Examples 1, 2, 3, 4, 8 and 9 as concrete examples of production process of a compound wherein Y is CONR⁷. Example 1 describes an example of production by Steps A and B. Example 2 describes an example of production by Step C. Example 3 describes an example of production by Step D. Example 8 describes an example of production by Steps A and B. In any of these examples, first, coupling carboxylic acid and amine is performed by Step A, and then Steps B, C, D are performed.

Examples 4 and 9 disclose processs in which the last step is a coupling step, although they are not included in the above general production process. Example 4 describes a synthesis process shown below. The objective product is obtained in 70% yield.

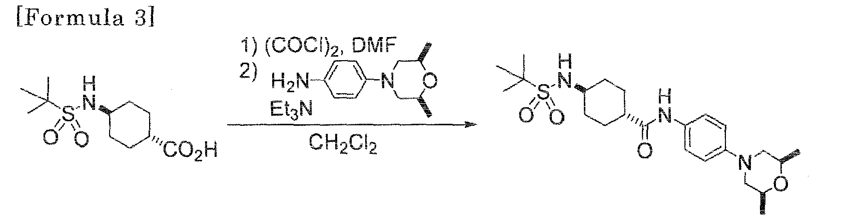

Example 9 discloses a synthesis process shown below. There is no description about yield.

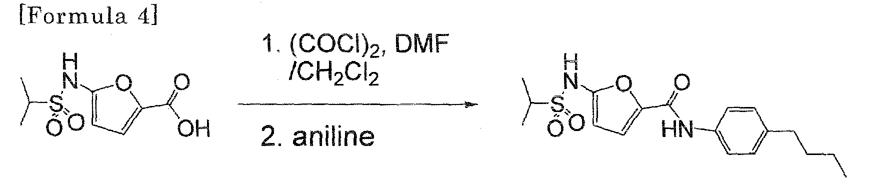

As a synthesis process using a compound represented by the formula: 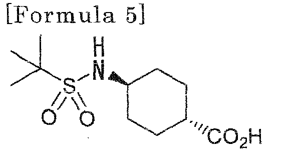 as a starting material, Patent Document 2 discloses the following synthesis process besides Example 4 in Patent Document 1. 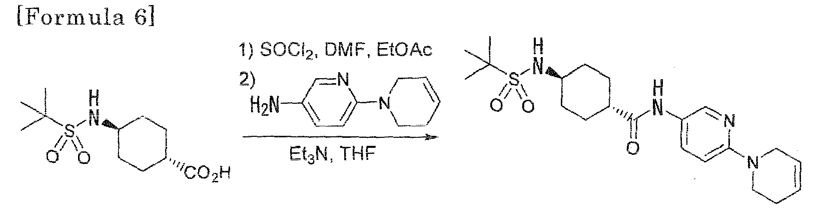 In the above reaction, the objective product is obtained in 81.4% yield. 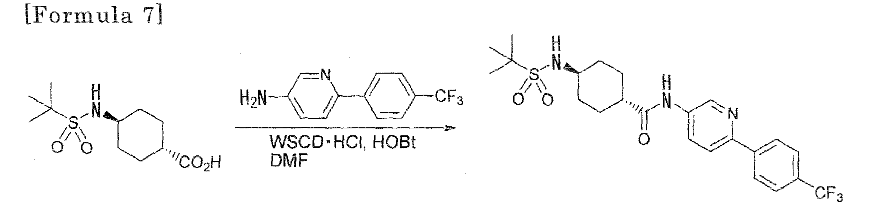 In the above reaction, the objective product is obtained in 33.6% yield. 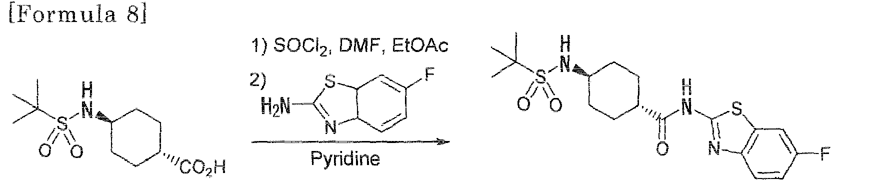 In the above reaction, the objective product is obtained in 55.8% yield.

The above cyclohexane derivative is disclosed also in Patent Document 3, Patent Document 4 and Patent Document 5. Also general description in synthesizing a compound having NPYY5 receptor antagonism is found. However, a concrete example of synthesis of a compound having NPYY5 receptor antagonism is not described.

The above Patent Documents 1 to 5 describe a compound represented by the formula (IV): 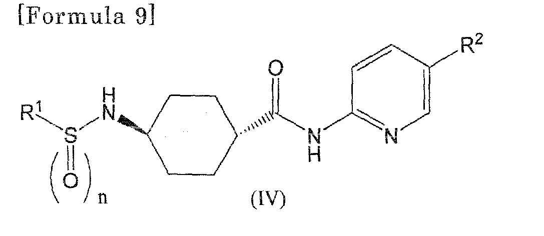 wherein R¹ is substituted or unsubstituted alkyl, n is 1 or 2, and R² is substituted or unsubstituted alkyl, however, a concrete synthesis process is not disclosed.

Patent Document 1 describes that melting point of the compound of Example No. Ia-178 is 223 to 224°C.

### [Prior Art Documents]

### [Patent Documents]

[Patent Document 1] WO2001/037826
[Patent Document 2] WO2003/076374
[Patent Document 3] JP2005-255630
[Patent Document 4] WO2006/106800
[Patent Document 5] WO2008/038640

### [Disclosure of Invention]

### [Problems to be solved by the Invention]

The present invention provides a process of producing a compound having NPYY5 receptor antagonism and a useful crystal, and particularly an efficient process for producing a compound described in Example No. Ia-178 of Patent Document 1.

### [Means for solving the problem]

As a result of diligent effort, the present inventors have found a process for producing a compound having NPYY5 receptor antagonism and a useful crystal, and have accomplished the following invention.
(1)
   A process for producing a compound represented by the formula (IV): 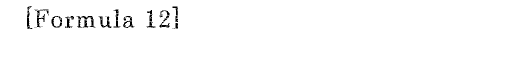 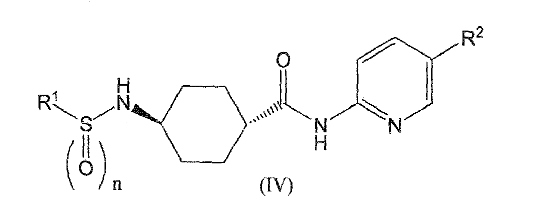 wherein R¹ is substituted or unsubstituted alkyl, R² is substituted or unsubstituted alkyl, and n is 1 or 2, its salt or a solvate thereof, which comprises reacting a compound represented by the formula (II): 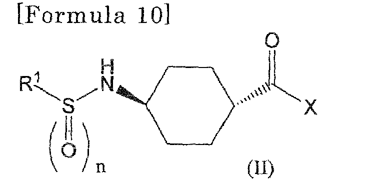 wherein R¹ and n are as defined above, and X is a leaving group, its salt or a solvate thereof, and
   a compound represented by formula (III): 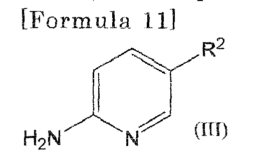 wherein R² is as defined above, its salt or a solvate thereof.
(2)
   The process according to the above (1), comprising a step of obtaining a compound represented by the formula (II): 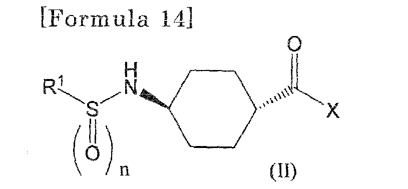 wherein R¹ is substituted or unsubstituted alkyl, n is 1 or 2, and X is halogen, its salt or a solvate thereof by reacting a compound represented by the formula (I) 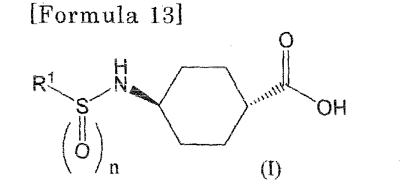 wherein R¹ and n are as defined above, its salt or a solvate thereof and a halogenating agent.
(3)
   The process according to the above (2), wherein the halogenating agent is selected from phosphorus oxychloride, phosphorus pentachloride, oxalyl chloride, and thionyl chloride.
(4)
   The process according to any one of the above (1) to (3), comprising reacting a compound represented by the formula (II), its salt or a solvate thereof and a compound represented by the formula (III), its salt or a solvate thereof in the presence of a base.
(5)
   The process according to any one of the above (1) to (4), wherein toluene, pyridine, tetrahydrofuran, dimethylformamide, a mixed solvent thereof or an aqueous solvent thereof is used as a solvent.
(6)
   The process according to any one of the above (1) to (5), wherein R¹ is C2-C4 alkyl, R² is alkyl substituted with halogen, and n is 2.
(7)
   The process according to any one of the above (1) to (6), wherein R¹ is tert-butyl, R² is trifluoromethyl, n is 2, and a compound represented by the formula (IV) is a crystal having peaks at 2θ = 12.5±0.2, 16.8±0.2, 17.8±0.2, 19.0±0.2 degrees in a powder X-ray diffraction pattern.
(8)
   The process according to any one of the above (1) to (6), wherein R¹ is tert-butyl, R² is trifluoromethyl, n is 2, and a compound represented by the formula (IV) is a crystal having peaks at 2θ =12.5±0.2, 14.3±0.2, 16.8±0.2, 17.8±0.2, 18.4±0.2, 19.0±0.2, 21.6±0.2, 33.1±0.2 degrees in a powder X-ray diffraction pattern.
(9)
   A process for producing a crystal of a compound represented by the formula (IV): 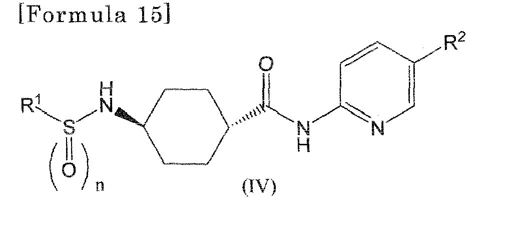 wherein R¹ is tert-butyl, R² is trifluoromethyl, and n is 2, having peaks at 2θ = 10.2±0.2, 17.1±0.2, 17.7±0.2, 26.5±0.2 degrees in a powder X-ray diffraction pattern, comprising recrystallizing a crystal of a compound represented by the formula (IV) having peaks at 2θ = 12.5±0.2, 16.8±0.2, 17.8±0.2, 19.0±0.2 degrees in a powder X-ray diffraction pattern.
(10)
   A process for producing a crystal of a compound represented by the formula (IV): 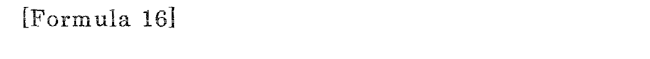 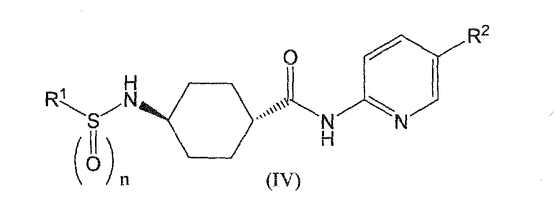 wherein R¹ is tert-butyl, R² is trifluoromethyl, and n is 2, having peaks at 2θ = 10.2±0.2, 14.5±0.2, 16.8±0.2, 17.1±0.2, 17.7±0.2, 18.3±0.2, 19.1±0.2, 20.2±0.2, 20.8±0.2, 26.5±0.2 degrees in a powder X-ray diffraction pattern, comprising recrystallizing a crystal of a compound represented by the formula (IV) having peaks at 2θ = 12.5±0.2, 14.3±0.2, 16.8±0.2, 17.8±0.2, 18.4±0.2, 19.0±0.2, 21.6±0.2, 33.1±0.2 degrees in a powder X-ray diffraction pattern.
(11)
   A process for producing a crystal of a compound represented by the formula (IV) having peaks at 2θ = 10.2±0.2, 17.1±0.2, 17.7±0.2, 26.5±0.2 degrees in a powder X-ray diffraction pattern, comprising obtaining a crystal of a compound represented by the formula (IV) having peaks at 2θ = 12.5±0.2, 16.8±0.2, 17.8±0.2, 19.0±0.2 degrees in a powder X-ray diffraction pattern by the process according to the above (7), and recrystallizing the obtained crystal.
(12)
   A process for producing a crystal of a compound represented by the formula (IV) having peaks at 2θ = 10.2±0.2, 14.5±0.2, 16.8±0.2, 17.1±0.2, 17.7±0.2, 18.3±0.2, 19.1±0.2, 20.2±0.2, 20.8±0.2, 26.5±0.2 degrees in a powder X-ray diffraction pattern, comprising obtaining a crystal of a compound represented by the formula (IV) having peaks at 2θ = 12.5±0.2, 14.3±0.2, 16.8±0.2, 17.8±0.2, 18.4±0.2, 19.0±0.2, 21.6±0.2, 33.1±0.2 degrees in a powder X-ray diffraction pattern by the process according to the above (8), and recrystallizing the obtained crystal.
(13)
   A crystal of a compound represented by the formula (IV): 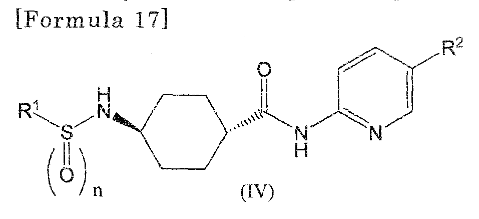 wherein R¹ is tert-butyl, R² is trifluoromethyl, and n is 2, having peaks at 2 θ =12.5±0.2, 16.8±0.2, 17.8±0.2, 19.0±0.2 degrees in a powder X-ray diffraction pattern.
(14)
   A crystal of a compound represented by the formula (IV): 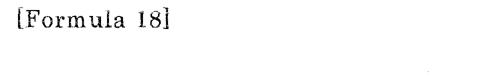 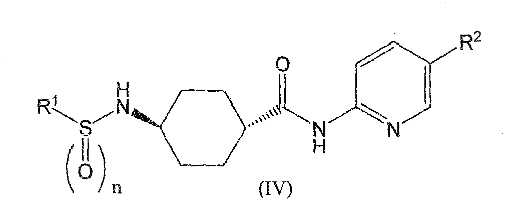 wherein R¹ is tert-butyl, R² is trifluoromethyl, and n is 2, having peaks at 2 θ =12.5±0.2, 14.3±0.2, 16.8±0.2, 17.8±0.2, 18.4±0.2, 19.0±0.2, 21.6±0.2, 33.1±0.2 degrees in a powder X-ray diffraction pattern.
(15)
   A crystal of a compound represented by the formula (IV): 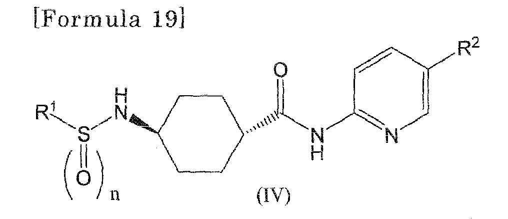 wherein R¹ is tert-butyl, R² is trifluoromethyl, and n is 2, having an endothermic peak at 219.6±2.0°C in a differential scanning calorimetry.
(16)
   A compound represented by the formula (II): 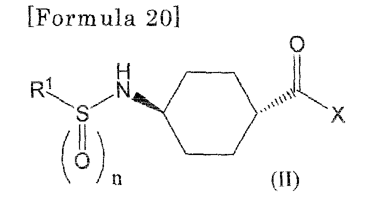 wherein R¹ is substituted or unsubstituted alkyl, n is 2, and X is halogen, its salt or a solvate thereof.
(A1)
   A process for producing a compound represented by the formula (IV): 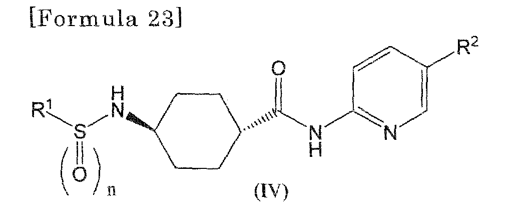 wherein R¹ is substituted or unsubstituted alkyl, R² is substituted or unsubstituted alkyl, and n is 1 or 2, its salt or a solvate thereof, which comprises reacting a compound represented by the formula (II): 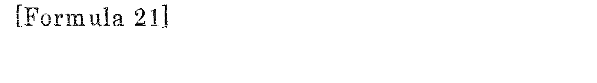 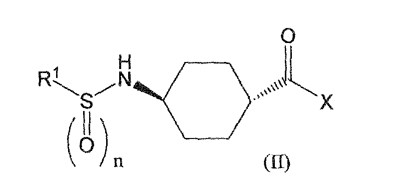 wherein R¹ and n are as defined above, and X is a leaving group, its salt or a solvate thereof, and
   a compound represented by formula (III): 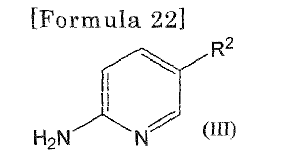 wherein R² is as defined above, its salt or a solvate thereof.
(A2)
   The process according to the above (A 1), comprising a step of obtaining a compound represented by the formula (II): 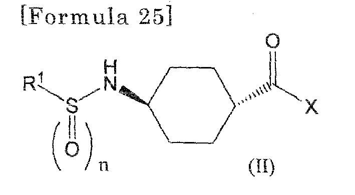 wherein R¹ is substituted or unsubstituted alkyl, n is 1 or 2, and X is halogen, its salt or a solvate thereof by reacting a compound represented by the formula (1): 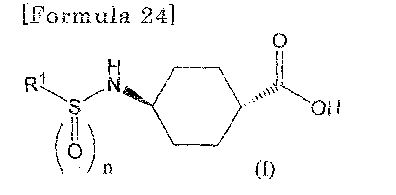 wherein R¹ and n are as defined above, its salt or a solvate thereof and a halogenating agent.
(A3)
   The process according to the above (A 2), wherein the halogenating agent is selected from phosphorus oxychloride, phosphorus pentachloride, oxalyl chloride, and thionyl chloride.
(A4)
   The process according to any one of the above (A 1) to (A 3), comprising reacting a compound represented by the formula (II), its salt or a solvate thereof and a compound represented by the formula (III), its salt or a solvate thereof in the presence of a base.
(A5)
   The process according to any one of the above (A 1) to (A 4), wherein toluene, pyridine, tetrahydrofuran, dimethylformamide, a mixed solvent thereof or an aqueous solvent thereof is used as a solvent.
(A6)
   The process according to any one of the above (A 1) to (A 5), wherein R¹ is C2-C4 alkyl, R² is alkyl substituted with halogen, and n is 2.
(A7)
   The process according to any one of the above (A 1) to (A 6), wherein R¹ is tert-butyl, R² is trifluoromethyl, n is 2, and a compound represented by the formula (IV) is a crystal having peaks at 2θ =12.5, 14.3, 16.8, 17.8, 18.4, 19.0, 21.6, 33.1 degrees in a powder x-ray diffraction pattern.
(A8)
   A process for producing a crystal of a compound represented by the formula (IV): 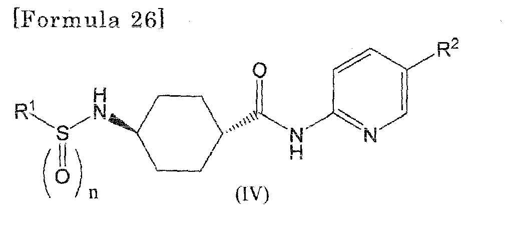 wherein R¹ is tert-butyl, R² is trifluoromethyl, and n is 2, having peaks at 28 = 10.2, 14.5, 16.8, 17.1, 17.7, 18.3, 19.1, 20.2, 20_{.}8, 26.5 degrees in a powder X-ray diffraction pattern, comprising recrystallizing a crystal of a compound represented by the formula (IV) having peaks at 2θ = 12.5, 14.3, 16.8, 17.8, 18.4, 19.0, 21.6, 33.1 degrees in a powder X-ray diffraction pattern.
(A9)
   A process for producing a crystal of a compound represented by the formula (IV) having peaks at 2θ = 10.2, 14.5, 16.8, 17.1, 17.7, 18.3, 19.1, 20.2, 20.8, 26.5 degrees in a powder X-ray diffraction pattern, comprising obtaining a crystal of a compound represented by the formula (IV) having peaks at 2θ = 12.5, 14.3, 16.8, 17.8, 18.4, 19.0, 21.6, 33.1 degrees in a powder X-ray diffraction pattern by the process according to the above (A7), and recrystallizing the obtained crystal.
(A10)
   A crystal of a compound represented by the formula (IV): 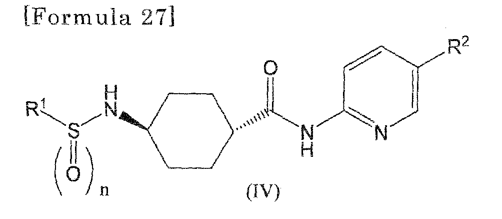 wherein R¹ is tert-butyl, R² is trifluoromethyl, and n is 2, having peaks at 2 θ =12.5, 14.3, 16.8, 17.8, 18.4, 19.0, 21.6, 33.1 degrees in a powder X-ray diffraction pattern.
(A11)
   A compound represented by the formula (II): 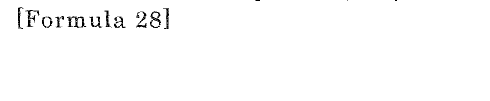 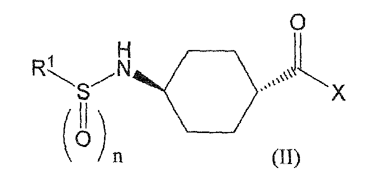 wherein R¹ is substituted or unsubstituted alkyl, n is 2, and X is halogen, its salt or a solvate thereof.

In production of active pharmaceutical ingredients of pharmaceuticals, recrystallization is performed for producing active pharmaceutical ingredients of high purity. In recrystallization, solubility of crude crystals in a recrystallizing solvent is very important.
The present inventors have found that a compound represented by the formula (IV): 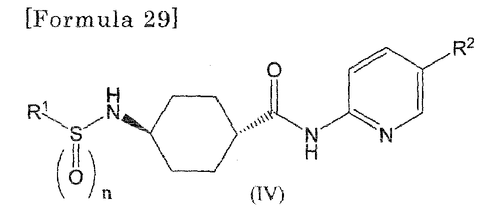 wherein R¹ is tert-butyl, R² is trifluoromethyl, and n is 2, has two crystal forms. One is a type I crystal having a melting point of 224.6±2.0°C, and the other is a type II crystal having a melting point of 219.6±2.0°C. Melting point of compound in Example No. la-178 of Patent Document 1 is described as being 223 to 224°C. Since melting point of type I crystal obtained by the present inventors was 224.6±2.0°C, it appears that a crystal described in Patent Document 1 is type 1.
The present inventors have found that type II crystal can be produced as shown in Examples 6 to 8.
The present inventors have found that, as shown in Example 12, a type II crystal of a compound represented by the formula (IV) (wherein R¹ is tert-butyl, R² is trifluoromethyl, and n is 2) exhibits higher solublitiy in a solvent than a type I crystal.
As shown in Example 9, it has been also found that a compound represented by the formula (IV) (wherein R¹ is tert-butyl, R² is trifluoromethyl, and n is 2) can be obtained as a type 1 crystal which is a stable form desired as active pharmaceutical ingredients of phermaceuticals by recrystallization of a type II crystal.

### [Effect of the Invention]

By using the present invention, it is possible to efficiently produce a compound having NPYY5 receptor antagonism, and to efficiently perform purification.

### [Brief Description of the Drawings]

[Fig. 1] Powder X-ray diffraction data of a type I crystal.
[Fig. 2] Powder X-ray diffraction data of a type II crystal.
[Fig. 3] Powder X-ray diffraction data of a type I crystal.
[Best Mode for Carrying Out the Invention]

In the following, meanings of terms used in the present specification will be explained.
"Alkyl" includes a straight or branched C1-C10 alkyl group, and example includes methyl, ethyl, n-propyl, isopropyl, n-butyl, isobutyl, sec-butyl, tert-butyl, n-pentyl, isopentyl, neopentyl, n-hexyl, isohexyl, n-heptyl, n-octyl, n-nonyl, n-decyl or the like. Preferable is C1-C6 or C1-C4 alkyl, and example includes methyl, ethyl, n-propyl, isopropyl, n-butyl, isobutyl, se-butyl, tert-butyl, n-pentyl, isopentyl, neopentyl, n-hexyl, or isohexyl.
As alkyl in R¹, C2-C4 alkyl is preferred, and example includes ethyl, n-propyl, isopropyl, n-butyl, isobutyl, see-butyl, or tert-butyl. Particularly preferred is tert-butyl.
As alkyl in R², C1-C4 alkyl is preferred. Example includes methyl, ethyl, n-propyl, isopropyl, n-butyl, isobutyl, sec-butyl, or tert-butyl. Particularly preferred is methyl.
Example of a substituent for "substituted or unsubstituted alkyl", includes halogen, alkoxy or the like. Such alkyl may be substituted with such a substituent at one to six position(s).
"Halogen" includes fluorine, chlorine, bromine or iodine.
Alkyl part in "alkoxy" is as defined in the "alkyl."
As R¹, unsubstituted alkyl is preferred.
As R², alkyl substituted with halogen is preferred. Particularly, as R², trifluoromethyl is preferred.
n is 1 or 2, and is preferably 2.
"Leaving group," includes any substituent that will leave at the time of condensation between carboxylic acid and amine without any particular limitation. Example includes halogen, acyloxy (for example, acetyloxy, benzoyloxy or the like), substituted or unsubstituted alkyl, sulfonyloxy (for example, methane sulfonyloxy, trifluoromethane sulfonyloxy or the like), substituted or unsubstituted benzene sulfonyloxy (for example, paratoluene sulfonyloxy, orthonitrobenzene sulfonyloxy or the like), N,N'-di-eyelohexylcarbamimidoyloxy, N,N'-diisopropyicarbamimidoyloxy, and (N-(3-(dimethylamino)propyl)-N'-ethylearbamimidoyloxy or the like. Halogen is preferred, and calor is particularly preferred.
"Salt," includes salt of inorganic acid such as hydrochloric acid, sulfuric acid, nitric acid or phosphoric acid; salt of organic acid such as acetic acid, formic acid, p-toluene sulfonic acid, methane sulfonic acid, oxalic acid or citric acid; salt of organic base such as ammonium, trimethyl ammonium or triethyl ammonium; salt of alkaline metal such as sodium or potassium; salt of alkaline earth metal such as calcium or magnesium or the like.
"Solvate," includes hydrate, alcohol solvate or the like of compound or its salt. Example includes 0.5 hydrate, monohydrate, dehydrate or the like.

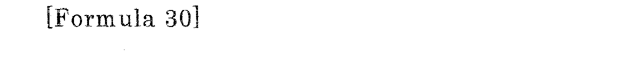

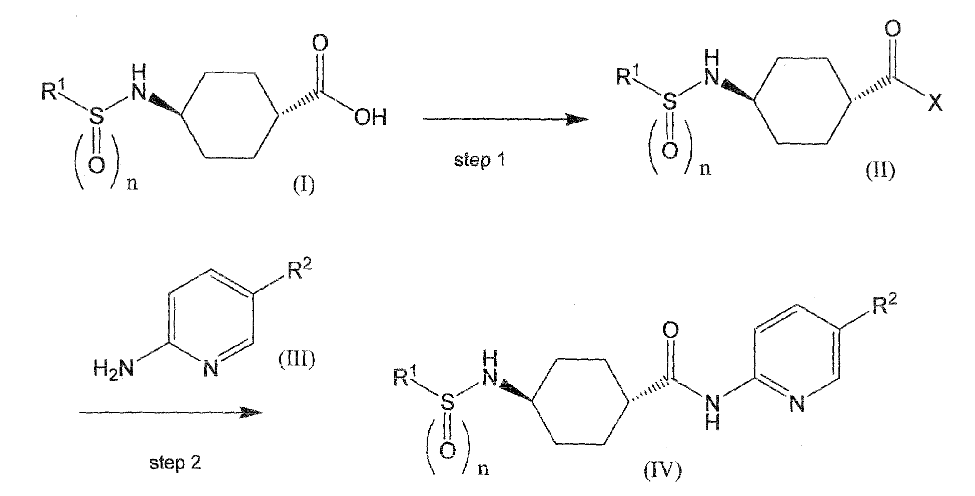

wherein R¹ is substituted or unsubstituted alkyl, n is 1 or 2, X is a leaving group, and R² is substituted or unsubstituted alkyl.

### Step 1

Step 1 is a process for preparing a compound represented by the formula (II) which comprises reacting a compound represented by the formula (I) with a halogenating agent.
Example of a halogenating agent includes phosphorus oxychloride, phosphorus pentachloride, oxalyl chloride, thionyl chloride, sulfuryl chloride, dichlorotriphenyl phosphorane or the like. Particularly preferred is phosphorus oxychloride, phosphorus pentachloride, oxalyl chloride, or thionyl chloride.
As a reaction solvent, dichloromethane, toluene, benzene, tetrahydrofuran, chloroform, dimethylformamide, acetonitrile, diethyl ether, benzene, xylene, cyclohexane, hexane, ethyl acetate, butyl acetate, pentane, heptane, dioxane, acetone, water or a mixed solvent, thereof may be used. Particularly preferred is toluene, tetrahydrofuran, or dimethylformamide.
Reaction may be performed at about 0 to 100°C, preferably at room temperature to 60°C. Reaction time is 0.5 hour to 20 hours, and preferably 1 to 10 hour(s).
After end of reaction, the reaction solution is cooled and filtered to collect a compound represented by the formula (II) which is ready for use in next Step 2. Alternatively, Step 2 may be continuously performed without collection of a compound represented by the formula (II) by filtering.
In the above Step 1, compounds represented by the formula (II) having various leaving groups may be synthesized by using reagents other than the halogenating agent. For example, by letting acyl halide react with a compound represented by the formula (I) in the presence of a base, a compound represented by the formula (II) having acyloxy as "leaving group" can be produced. By letting substituted or unsubstituted alkylsulfonyl halide react with a compound represented by the formula (I) in the presence of a base, a compound represented by the formula (II) having substituted or unsubstituted alkylsulfonyloxy as "leaving group" can be produced. The same applies to other cases.
As a compound represented by the formula (II): 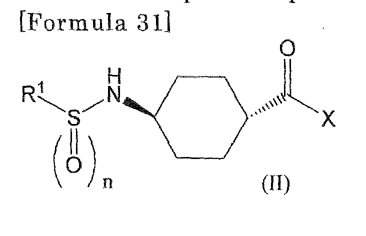 wherein R¹ is substituted or unsubstituted alkyl, n is 1 or 2, and X is a leaving group, a compound wherein R¹ is substituted or unsubstituted alkyl, n is 2 and X is halogen is preferred. Further, a compound wherein R¹ is tert-butyl, n is 2, X is halogen, especially chloro, is preferred.

### Step 2

Step 2 is a process for preparing a compound represented by the formula (IV) which comprises reacting a compound represented by the formula (II) with a compound represented by the formula (III).
This reaction may be performed in the presence of a base. Example of a base includes pyridine, triethylamine, dimethylaminopyridine, morpholine, N-methylmorpholine, diisopropylethylamine or the like. Pyridine may be used as a solvent.
As a reaction solvent, the solvent that is used in Step 1 may be used. Particularly preferred is toluene, tetrahydrofuran, or dimethylformamide.
Reaction may be performed at about 0 to 100°C, preferably at room temperature to 60°C. Reaction time is 0.5 hour to 20 hours, and preferably 1 to 10 hour(s).

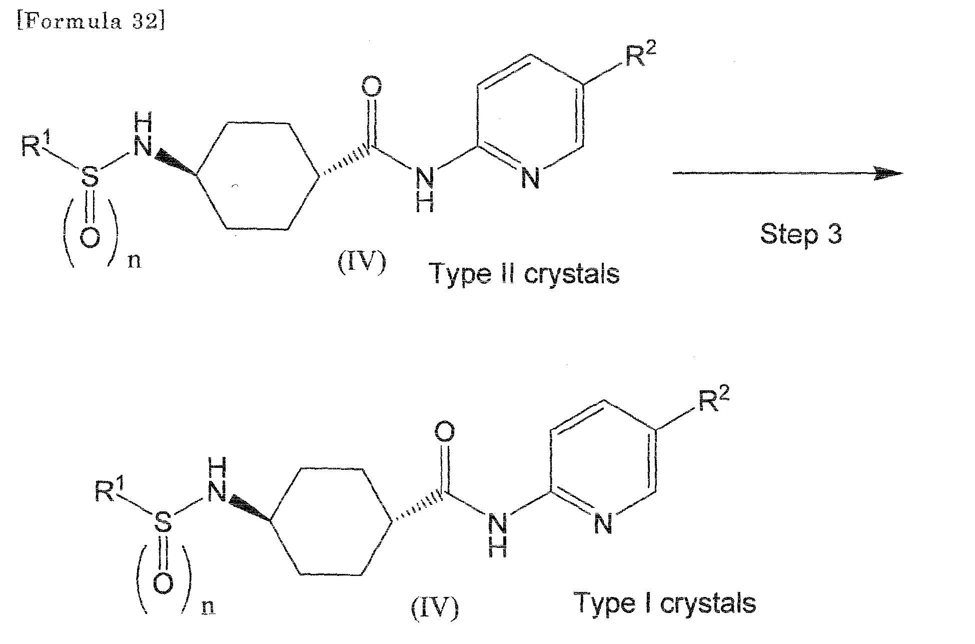

wherein R¹ is tert-butyl, R² is trifluoromethyl, and n is 2.

### Step 3

Step 3 is a step of obtaining type I crystal from type II crystal. A type I crystal has low solubility and requires a large amount of solvent for recrystallization. A type II crystal has higher solubility than a type I crystal, and is very useful for purifying a compound represented by the formula (IV).
Recrystallization from a type II crystal to a type I crystal may be performed by using acetone, dichloromethane, methanol, ethyl acetate, tetrahydrofuran, dimethylformamide, acetonitrile or a mixed solvent thereof. A type II crystal is dissolved in a solvent by heating to about 50 to 100°C, and then allowed to crystallize. Crystallization may be performed after increasing the concentration of the compound represented by the formula (IV) by concentrating under reduced pressures. Also crystallization may be performed by cooling. Also, crystallization may be performed by adding a poor solvent (solvent in which solubility of a compound represented by the formula (IV) is small). Also these crystallization techniques may be used in combination. For example, dissolution in acetone is followed by concentration and addition of water to achieve crystallization. As water added, deionized water is preferred.
A compound represented by the formula (IV) (wherein R¹ is tert-butyl, R² is trifluoromethyl, and n is 2) has two crystal forms. Spectrum data thereof will be described below.

### Type I crystal

### Melting point (DSC) is 224.6 ± 2.0°C.

A characteristic peak is observed at 1708±2 cm⁻¹ in IR spectrum.
Peaks are observed at 2θ = 10.2±0.2,14.5±0.2, 16.8±0.2, 17.1±0.2, 17.7±0.2, 18.3±0.2, 19.1±0.2, 20.2±0.2, 20.8±0.2, 26.5±0.2 degrees in a powder X-ray diffraction pattern.

### Type II crystal

### Melting point (DSC) is 219.6 ± 2.0°C.

A characteristic peak is observed at 1686±2 cm⁻¹ in IR spectrum.
Peaks are observed at 2θ = 12.5±0.2, 14.3±0.2, 16.8±0.2, 17.8±0.2, 18.4±0.2, 19.0±0.2, 21.6±0.2, 33.1±0.2 degrees in a powder X-ray diffraction pattern.
By measuring spectrum data of compound, it is possible to identify whether the obtained crystal is a type I crystal or type II crystal.

Numerical values described in the present specification and claims are approximate values unless otherwise referred. Variation in numerical value is attributed to apparatus calibration, apparatus error, purity of substance, crystal size, sample size and other factors.

As for DISC, it is known that the observed temperature can depend on temperature change speed, employed sample preparation techniques, and a particular apparatus. Since an error range in DSC thermograph is about ± 2.0°C, it should be understood that the above value of melting point (DSC) includes values within a range of about ± 2.0°C. In determination of identity of crystals, an overall pattern is important which can change more or less depending on the measurement condition.
Melting point (DSC) of type I crystal is 224.6 ± 2.0°C, preferably 224.6 ± 1.0°C, and more preferably 224.6 ± 0.6°C.
Melting point (DSC) of type II crystal is 219.6 ± 2.0°C, preferably 219.6 ± 1.0°C, and more preferably 219.6 ± 0.6°C.

### Examples

The present invention is further explained by the following Examples, which are not intended to limit the scope of the present invention.
DMF: dimethylformamide
THF: tetrahydrofuran
WSC: water-soluble carbodiimide (1-ethyl-3-(3-dimethylaminopropyl) carbodiimide hydrochloride)
HOBt: 1-hydroxybenzotriazole

### [Example 1]

### Production method of acid chloride (2)

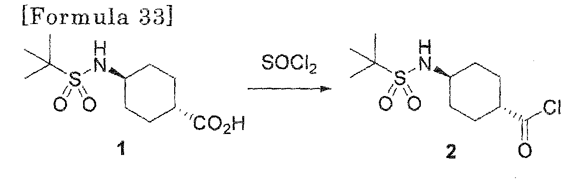

Under nitrogen gas flow, to a solution of 5.0 g of compound (1) in 20 mL of toluene was added 27.8 mg of DMF, and temperature was adjusted to 50°C. To the resultant solution was added 2.49 g of thionyl chloride dropwisely, and the reaction solution was stirred for 50 minutes. After stirring under ice cooling, deposited solid was collected by filtering under nitrogen gas flow and washed with toluene, to obtain 5.39 g of acid chloride (2) (yield 101%).
¹H NMR (300 MHz, CDCl₃): δ1.14-1.44 (2H, m), 1.39 (9H, s), 1.50-1.74 (4H, m), 2.14-2.31 (4H, m), 2.66 (1H, tt, J = 3.3, 12.0 Hz), 3.18-3.35 (1H, m), 3.80 (1H, d, J = 8.7 Hz).
IR (ATR) : 3293, 1803, 1364 cm⁻¹

### [Example 2]

### Production method of compound (4) by coupling between acid chloride (2) and aminotrifluoromethylpyridine (3)

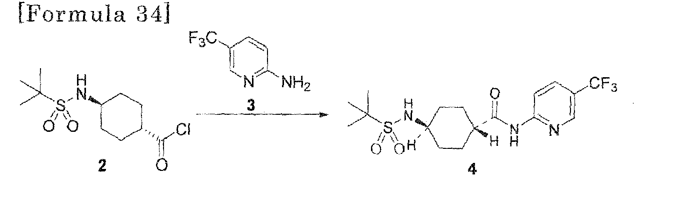

To 1.09 g of acid chloride (2), 5.5 mL of toluene was added, and a solution of 677 mg of aminotrifluoromethylpyridine (3) and 330 mg of pyridine in 0.5 mL of toluene at room temperature was added dropwisely over 15 minutes, and the reaction solution was stirred for 1 hour at 30°C. To this reaction solution, 5 mL of water was added dropwisely. After collecting the precipitated crystals by filtration, 1.45 g (93.4 %) of white crystals (4) was obtained.
¹H NMR (300 MHz, DMSO-d₆): δ1.27 (s, 9H), 1.25-1.75 (m, 4H), 1.82-2.00 (m, 4H), 2.40-2.54 (m, 1H), 3.00-3.15 (m, 1H), 6.79 (d, 1H, J = 8.4 Hz), 8.10-8.18 (m, 1H), 8.28 (d, 1H, J = 8.7 Hz), 8.69 (s, 1H), 10.84 (s, 1H)

### [Example 3]

### Production method of compound (4) using phosphorus oxychloride

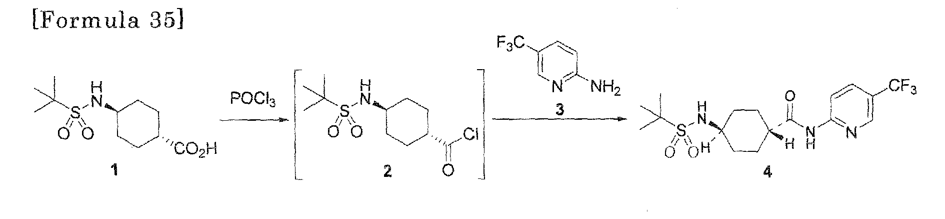

Under nitrogen gas flow, a solution of 666 mg of pyridine in 1 mL of acetonitrile was added to a solution of 1.0 g of compound (1) in 3 mL of acetonitrile under ice cooling, and continuously 13.9 mg of DMF was added. To the resultant solution, a solution of 641 mg of phosphorus oxychloride in 1 my of acetonitrile was added dropwisely over 5 minutes under ice cooling, and stirred for 1 hour. To the resultant solution, a solution of 677 mg of aminotrifluoromethylpyridine (3) in 1.5 mL of acetonitrile was added dropwisely over 4 minutes under ice cooling, and the reaction solution was stirred for 30 minutes, and then stirred at 30°C for 1.5 hours.
To this solution, 7 mL of water was added dropwisely. The mixture was filtrated, and crystals were washed with 5 mL or water. 1.14 g (73.7 %) of (4) was obtained in white crystals.

### [Example 4]

### Production method of compound (4) using oxalyl chloride

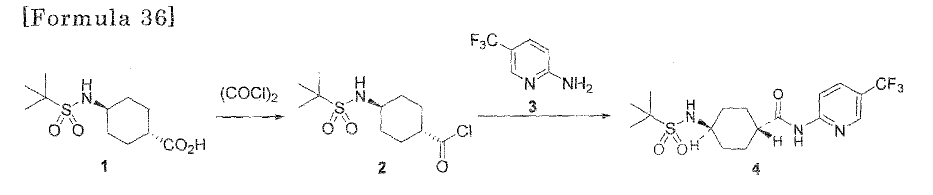

Under nitrogen gas flow, to a solution of 1.0 g of compound (1) in 4 mL of methylene chloride was added 13.9 mg of DMF under ice cooling. After adding dropwisely a solution of 530 mg of oxalyl chloride in a 1.5 mL of methylene chloride at the same temperature, and stirring for 1 hour, the solution was adjusted to be at room temperature, and stirred for 3 hours. Solvent was distilled off from the solution under reduced pressures, and 1.21 g of acid chloride (2) was obtained. This compound (2) was added with 3.5 mL of toluene, the temperature was adjusted to 30°C, and a solution of 677 mg of aminotrifluoromethylpyridine (3) and 331 mg of pyridine in 1.5 mL of toluene was added dropwisely over 10 minutes, and the reaction solution was stirred for 50 minutes. To this solution, 5 mL of water was added dropwisely, and precipitated crystals were collected by filtration, to obtain 1.42 g (91.5 %) of (4) in white crystals.

### [Example 5]

### Production method of compound (4) using phosphorus pentachloride

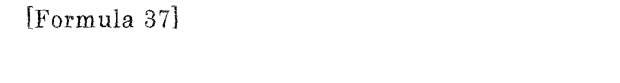

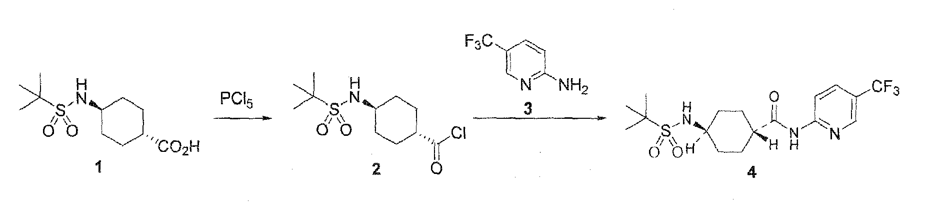

Under nitrogen gas flow, to a solution of 1.0 g of compound (1) in 5 mL of methylene chloride was added 871 mg of phosphorus pentachloride under ice cooling and the reaction solution was stirred for 1 hour. Solvent was distilled off from the solution under reduced pressures, to obtain 1.09 g of acid chloride (2). To this acid chloride (2), 5.5 mL of toluene was added, and a solution of 677 mg of aminotrifluoromethylpyridine (3) and 330 mg of pyridine in 0.5 mL of toluene was added dropwisely over 15 minutes at room temperature and the reaction solution was stirred for 1 hour after adjustment of temperature to 30°C. To this reaction solution, 5 mL of water was added dropwisely. Precipitated crystals were collected by filtration, to obtain 1.45 g (93.4 %) of (4) in white crystals.

### [Example 6]

### Production method of compound (4) using thionyl chloride (solvent: toluene)

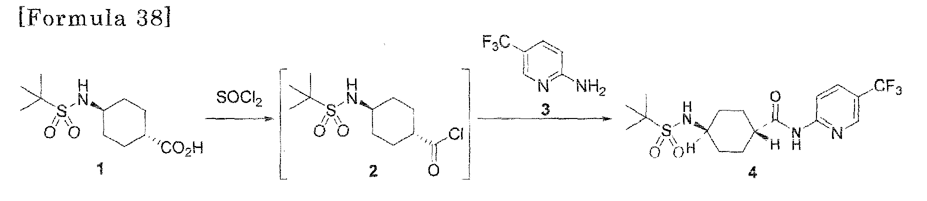

Under nitrogen gas flow, 0.05 kg of DMF was added to a solution in which 30.5 kg of toluene was added to 8.8 kg of compound (1). Temperature of the solution was adjusted to 50°C, and 4.4 kg of thionyl chloride was added dropwisely over 63 minutes at 48°C to 51°C. After stirring for 65 minutes at the same temperature, the temperature was adjusted to 30°C, and a solution of 5.4 kg of aminotrifluoromethylpyridine (3) and 5.8 kg of pyridine in 3.8 kg of toluene was added dropwisely over 40 minutes at 30°C to 37°C, followed by washing with 3.8 kg of toluene. After stirring this solution for 60 minutes at 30 °C to 37°C, 26.4 kg of water was added dropwisely at 30°C to 32°C over 10 minutes. After stirring this solution at 31°C to 32°C for 70 minutes, crystals were collected by filtration, and washed twice with 6.9 kg of isopropanol. 15.0 kg (dry weight 12.4 keg, 91.2 %) of compound (4) was obtained in white crystals. IR spectrum of white crystals of compound (4) was measured, and a characteristic peak was observed at IR 1686 cm (measured by KBr). The obtained crystals were type II crystals.

### [Example 7]

### Production method of compound (4) using thionyl chloride (solvent: THF)

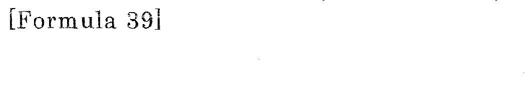

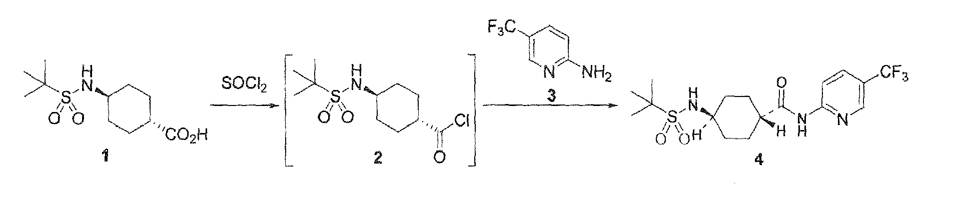

Under nitrogen gas flow, to a solution of 0.5 g of compound (1) in 2.0 mL of THF was added 2.8 mg of DMF under ice cooling. After adjusting the temperature of this solution to 50°C, a solution of 258 mg of thionyl chloride in 0.1 mL of THF was added and the reaction solution was stirred for 1.5 hours. Temperature of reaction solution was adjusted to 30°C, and a solution of 339 mg of aminotrifluoromethylpyridine (3) and 330 mg of pyridine in 0.5 mL of THF was added dropwisely over 8 minutes, and the reaction solution was stirred for 3 hours at the same temperature, and then water was added. 647 mg (87.2%, quantitative value) of objective product (4) was obtained. An organic layer was separated and filtered, and then washed with THF solution, and the solvent was distilled off from the obtained solution under reduced pressures, to result in precipitation of crystals. These crystals were collected by filtration, and 406 mg (52.5 %) of compound (4) was obtained in white crystals. IR spectrum of white crystals of compound (4) was measured, and a characteristic peak was observed at IR 1686 cm⁻¹ (measured by KBr). The obtained crystals were type II crystals.

### [Example 8]

### Production method of compound (4) using thionyl chloride (solvent: DMF)

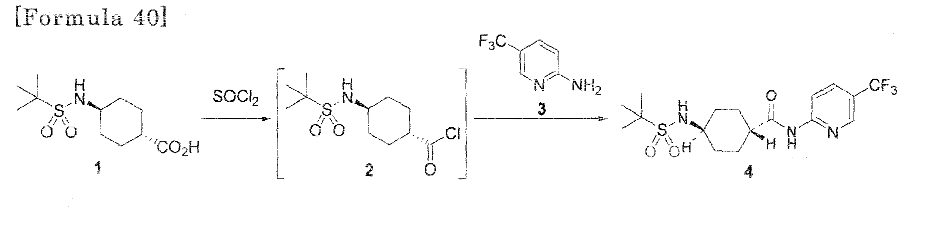

Under nitrogen gas flow, temperature of a solution of 0.5 g of compound (1) in 2.0 mL of DMF was adjusted to 50°C. To this solution, a solution of 249 mg of thionyl chloride in 0.1 mL of DMF was added and the reaction solution was stirred for 1.5 hours. Temperature of the reaction solution was adjusted to 30°C, and a solution of 339 mg of aminotrifluoromethylpyridine (3) and 330 mg of pyridine in 0.5 mL of DMF was added dropwisely over 8 minutes, and stirred for 4 hours at the same temperature. 530 mg (68.5 %, quantitative value) of objective product (4) was generated. To this solution, 2 mL of water was added dropwisely and precipitated crystal was collected by filtration and dried under reduced pressures: 500 mg (64.6 %) of compound (4) was obtained in white crystals. IR spectrum of white crystals of compound (4) was measured, and a characteristic peak was observed at IR 1686 cm⁻¹ (measured by KBr). The obtained, crystals were type II crystals.

### [Example 9]

### Recrystallization from type II crystals to type I crystals

Under nitrogen gas flow, to 15.0 kg of type II crystals of compound (4) was added 150.3 kg of acetone , and crystals were dissolved at 50°C, subjected to dust removal and filtration, and then washed with 10.7 kg of acetone. After concentrating this solution at normal pressure, 68.1 kg of deionized water having subjected to dust removal and filtration was added dropwisely at 52°C to 54°C. The obtained suspension was stirred for 165 minutes at 10°C to 20°C.
Precipitated crystals were collected by filtration, and crystals were washed twice with 12.1 kg of aqueous solution of acetone, and dried under reduced pressures, to give 11.8 kg of type I crystals of compound (4).
IR. spectrum was measured, and crystals (type II crystals) of compound (4), being a starting material, showed a characteristic peak at IT 1686 cm⁻¹ (measured by KBr). Crystals (type I crystals) of compound (4), being a product, showed a characteristic, peak at. IR 1708 cm⁻¹ (measured by KBr).

### [Example 10]

### Powder X-ray structure analysis data

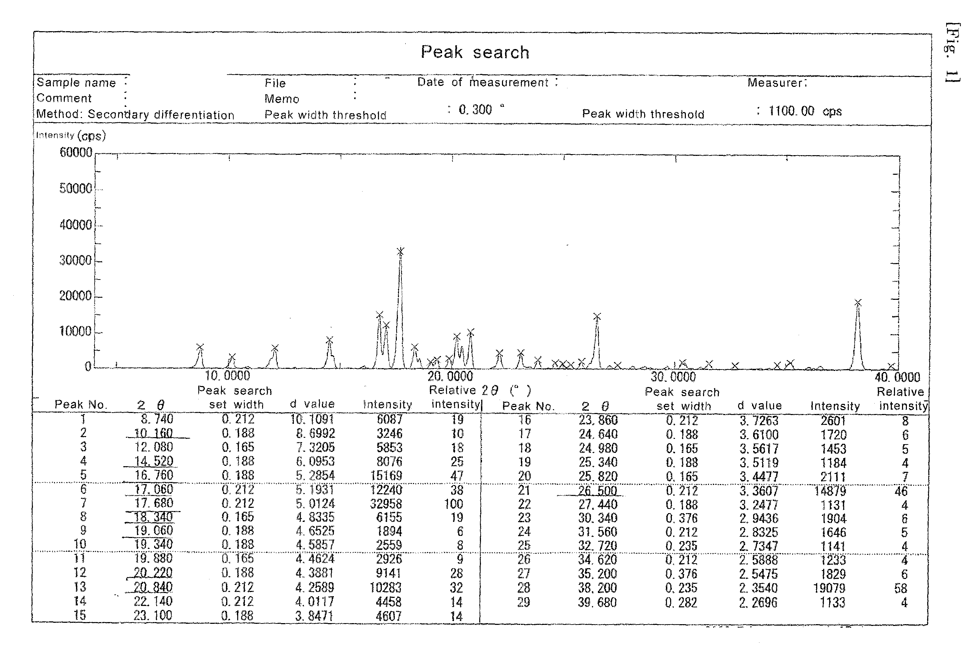

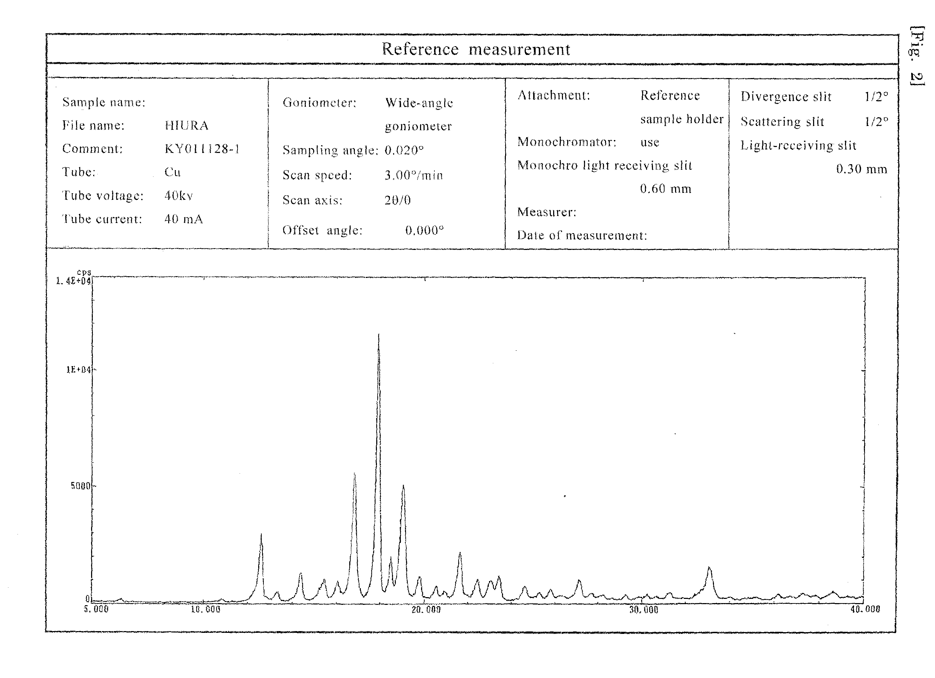

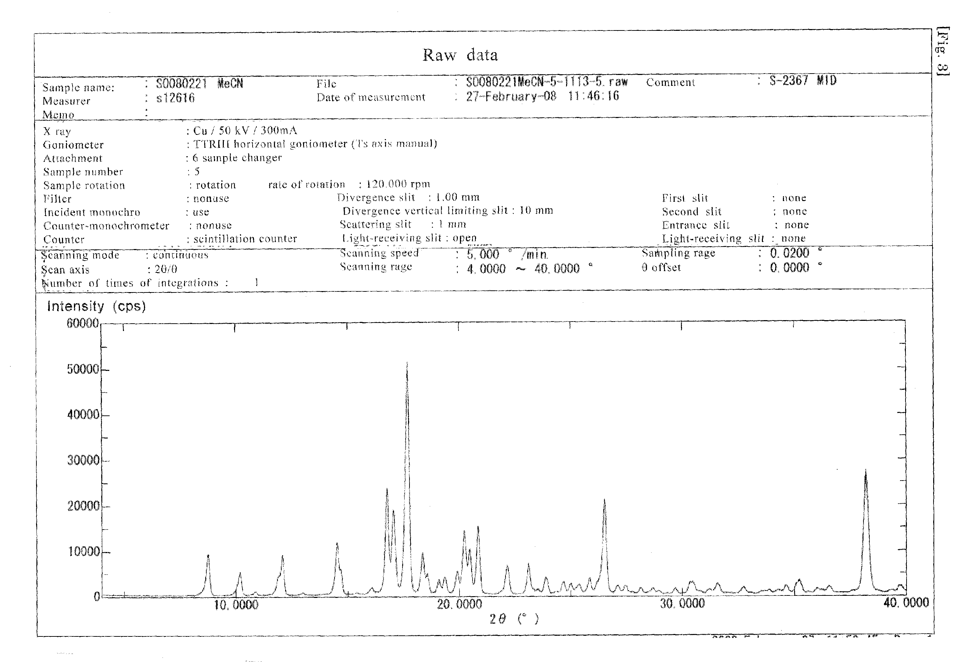

For a compound represented by the above (4), powder X-ray diffraction patterns of type I crystals and type II crystals were measured. As a result, crystals showing a peak at 1708 cm⁻¹ in IR spectrum were crystals that show peaks at 2θ =10.2, 14.5, 16.8, 17. 1, 17.7, 18.3, 19.1, 20.2, 20.8, 26.5 degrees (type I crystals). Crystals showing a peak at 1686 cm⁻¹ in IR spectrum were crystals that show peaks at 2θ = 12.5, 14.3, 16.8, 17.8, 18.4, 19.0, 21.6, 33.1 degrees (type II crystals). Fig. 1 and Fig. 3 show powder X-ray data of type I crystals, and Fig. 2 shows powder X-ray data of type II crystals.

### [Example 11]

### Melting point data (DSC)

Melting points of the type I crystals and type II crystals were measured. As a result, melting point of type I crystals was 224.6°C. Melting point of type II crystals was 219.6°C.

### [Example 12]

### Comparison of solubility of type I and type II in various solvents.

Solubility of type I and type II crystals in various solvents were measured, and the results are shown below. Solubility of type II crystals was determined by adding crystals gradually to each solvent, and measuring and calculating a dissolved amount.

**[Table 1]**

| Solvent | Solubility (wt%)/25°C | |
|---|---|---|
| | Type crystals | Type II crystal s |
| Dichloromethane | 1.02 | 2.46 |
| Toluene | 0.05 | 0.14 |
| Methanol | 1.23 | 4.80 |
| Acetone | 4.23 | 13.10 |
| EtOAc | 1.48 | 4.51 |
| THF | 7.25 | 24.06 |
| DMF | 21.91 | 33.00 |
| Acetonitrile | 1.22 | 2.61 |

### Comparative example 1

### Working example of coupling method by WSC/HOBt

Coupling experiment was performed by using a commonly used condensing agent. WSC/HOBt used as a coupling reagent in the following experiment is a reagent commonly used in a coupling method between carboxylic acid and amine. 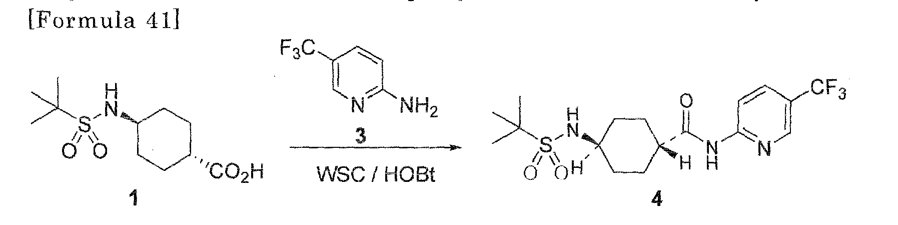 Under nitrogen gas flow, to a solution of 500 mg of compound (1) and 323 mg of aminotrifluoromethylpyridine (3) in 5 mL of DMF were added 364 mg of WSC and 307.8 mg of HOBt at -10°C, and the reaction solution was washed with 4 mL of DMF. Reaction temperature was raised to 0°C, and the reaction solution was stirred for 2 hours, and then stirred at room temperature for 20 hours. This reaction solution was quantified, and 2.8% of objective product (4) was generated.

### [Example 13]

### (XRPD)

### Measurement of powder X-ray diffraction pattern

### <Measurement condition of type I crystals>

Powder X-ray diffraction measurement of crystals obtained in each Example was performed under the following measurement condition according to a powder X-ray diffraction measurement method described in general test procedures of japanese Pharmacopoeia.
(Apparatus)
RINT TTR III available from Rigaku Corporation
(Operation method)
For a sample, measurement was performed under the following condition.

### Measurement method: reflection method

Type of optical source: Cu tube
Employed wavelength: CuKα ray
Tube current: 300 mA
Tube voltage: 50 Kv
Sample plate: aluminum (diffraction peak derived from aluminum 2θ = 38.2°) Incident angle of X-ray: 4° to 40°

### <measurement condition of type 11 crystals>

### (Apparatus)

RINT 1100 available from Rigaku Corporation

### (Operation method)

For a sample, measurement was performed under the following condition.

### Measurement method: reflection method

Type of optical source: Cu tube
Employed wavelength: CuKα ray
Tube current: 40 mA
Tube voltage: 40 Kv
Sample plate: glass
Incident angle of X-ray: 5° and 40°

### [Example 14]

### (R)

### Measurement of infrared absorption spectrum

Measurement of infrared absorption spectrum of crystals obtained in each Example was performed under the following condition.
(Apparatus)
Type MAGNA560 available from Thermo Fisher Scientific Inc. (previously: Thermo Electron Co., Ltd.)

### (Operation method)

For a sample, measurement was performed under the following condition.

### Measurement method: ATR method (KBr)

Resolution: 2 (cm⁻¹)
Detector: DTGS detector
Number of times of integrations: 32

### [Example 15]

### (DSC)

### Measurement of DSC data

### <measurement condition of type I crystals>

About 1.131 mg of crystals obtained in the above Example was weighed, charged into an aluminum pan, simply sealed and subjected to measurement. Measurement condition is as follows.

### (Measurement condition)

Apparatus: Rigaku Industrial Corporation Thermo Plus DSC8230L
Measurement range: 130-250°C
Rate or temperature rise: 10°C/min.
Atmosphere: N₂ 100 mL/min.

### <Measurement condition of type I I crystals>

About 0.976 mg of crystals obtained in the above Example was weighed, charged into an aluminum pan, simply sealed and subjected to measurement. Measurement condition is as follows.

### (Measurement condition)

Apparatus: Rigaku Industrial Corporation Thermo Plus DSC8230L
Measurement range: 130-250°C
Rate or temperature rise: 10°C/min.
Atmosphere: N₂ 100 mL/min.

### [Industrial applicability]

By using the present invention, it is possible to efficiently produce a compound having NPYY5 receptor antagonism, and to efficiently perform purification.

## Claims

1. A process for producing a compound represented by the formula (IV): 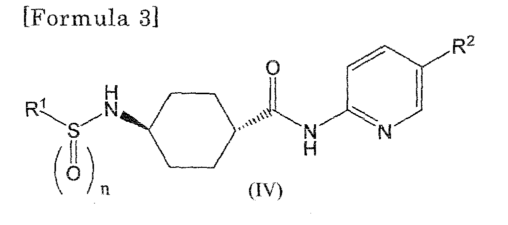 wherein R¹ is substituted or unsubstituted alkyl, R² is substituted or unsubstituted alkyl, and n is 1 or 2, its salt or a solvate thereof, which comprises reacting a compound represented by the formula (II): 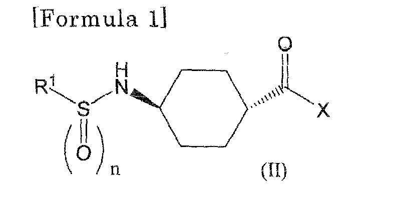 wherein R¹ and n are as defined above, and X is a leaving group, its salt or a solvate thereof, and
a compound represented by formula (III): 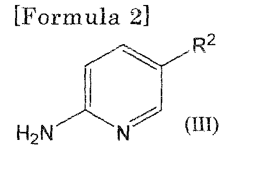 wherein R² is as defined above, its salt or a solvate thereof.

2. The process according to claim 1, comprising a step of obtaining a compound represented by the formula (II): 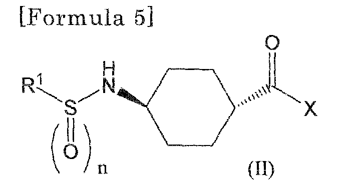 wherein R¹ is substituted or unsubstituted alkyl, n is 1 or 2, and X is halogen, its salt or a solvate thereof by reacting a compound represented by the formula (I): 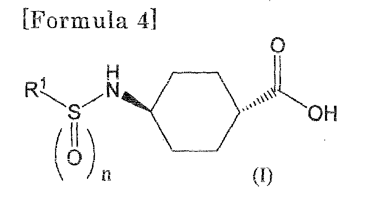 wherein R¹ and n are as defined above, its salt or a solvate thereof and a halogenating agent.

3. The process according to claim 2, wherein the halogenating agent is selected from phosphorus oxychloride, phosphorus pentachloride, oxalyl chloride, and thionyl chloride.

4. The process according to any one of claims 1 to 3, comprising reacting a compound represented by the formula (II), its salt or a solvate thereof and a compound represented by the formula (III), its salt or a solvate thereof in the presence of a base.

5. The process according to any one of claims 1 to 4, wherein toluene, pyridine, tetrahydrofuran, dimethylformamide, a mixed solvent thereof or an aqueous solvent thereof is used as a solvent.

6. The process according to any one of claims 1 to 5, wherein R¹ is C2-C4 alkyl, R² is alkyl substituted with halogen, and n is 2.

7. The process according to any one of claims 1 to 6, wherein R¹ is tert-butyl, R² is trifluoromethyl, n is 2, and a compound represented by the formula (IV) is a crystal. having peaks at 2θ = 12.5±0.2, 16.8±0.2, 17.8±0.2, 19.0±0.2 degrees in a powder X-ray diffraction pattern.

8. The process according to any one of claims 1 to 6, wherein R¹ is tert-butyl, R² is trifluoromethyl, n is 2, and a compound represented by the formula (IV) is a crystal having peaks at 2θ = 12.5±0.2, 14.3±0.2, 16.8±0.2, 17.8±0.2, 18.4±0.2, 19.0±0.2, 21.6±0.2, 33.1±0.2 degrees in a powder X-ray diffraction pattern.

9. A process for producing a crystal of a compound represented by the formula (IV): 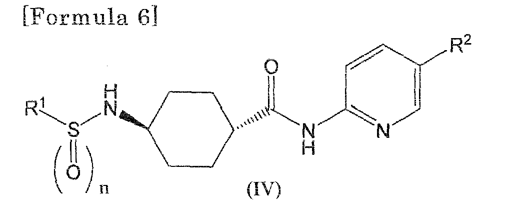 wherein R¹ is text-butyl, R² is trifluoromethyl, and n is 2, having peaks at 20 = 10.2±0.2, 17.1±0.2, 17.7±0.2, 26.5±0.2 degrees in a powder X-ray diffraction pattern, comprising recrystallizing a crystal of a compound represented by the formula (IV) having peaks at 20 = 12.5±0.2, 16.8±0.2, 17.8±0.2, 19.0±0.2 degrees in a powder X-ray diffraction pattern.

10. A process for producing a crystal of a compound represented by the formula (IV): 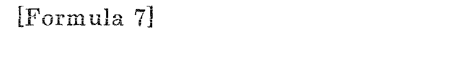 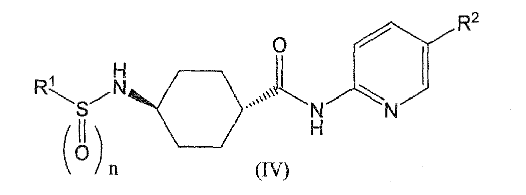 wherein R¹ is tert-butyl, R² is trifluoromethyl, and n is 2, having peaks at 2θ = 10.2±0.2, 14.5±0.2, 16.8±0.2, 17.1±0.2, 17.7±0.2, 18.3±0.2, 19.1±0.2, 20.2±0.2, 20.8±0.2, 26.5±0.2 degrees in a powder X-ray diffraction pattern, comprising recrystallizing a crystal of a compound represented by the formula (IV) having peaks at 2θ = 12.5±0.2, 14.3±9.2, 16.8±0.2, 17.8±0.2, 18.4±0.2, 19.0±0.2, 21.6±0.2, 33.1±0.2 degrees in a powder X-ray diffraction pattern.

11. A process for producing a crystal of a compound. represented by the formula (IV) having peaks at 2θ = 10.2±0.2, 17.1±0.2, 17.7±0.2, 26.5±0.2 degrees in a powder X-ray diffraction pattern, comprising obtaining a crystal of a compound represented by the formula (IV) having peaks at 2θ = 12.5±0.2, 16.8±0.2, 17.8±0.2, 19.0±0.2 degrees in a powder X-ray diffraction pattern by the process according to claim 7, and recrystallizing the obtained crystal.

12. A process for producing a crystal of a compound represented by the formula (IV) having peaks at 2θ= 10.2±0.2, 14.5±0.2, 16.8±0.2. 17.1±0.2, 17.7±0.2, 18.3±0.2, 19.1±0.2, 20.2±0.2, 20.8±0.2, 26.5±0.2 degrees in a powder X-ray diffraction pattern, comprising obtaining a crystal of a compound represented by the formula (IV) having peaks at 2θ = 12.5±0.2, 14.3±0.2, 16.8±0.2, 17.8±0.2, 18.4±0.2, 19.0±0.2, 21.6±0.2, 33.1±0.2 degrees in a powder X-ray diffraction pattern by the process according to claim 8, and recrystallizing the obtained crystal.

13. A crystal of a compound represented by the formula (IV): 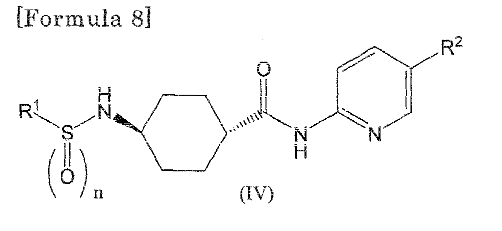 wherein R¹ is tert-butyl, R² is trifluoromethyl, and n is 2, having peaks at 2θ = 12.5±0.2, 16.8±0.2, 17.8±0.2, 19.0±0.2 degrees in a powder X-ray diffraction pattern.

14. A crystal of a compound represented by the formula (IV): 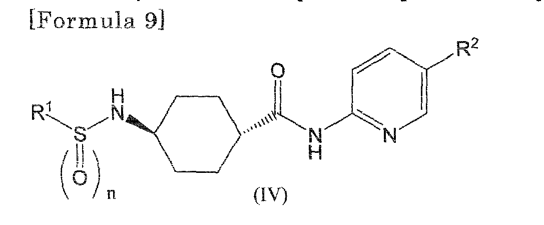 wherein R¹ is tert-butyl, R² is trifluoromethyl, and n is 2, having peaks at 2θ = 12.5±0.2, 14.3±0.2, 16.8±0.2, 17.8±0.2, 18.4±0.2, 19.0±0.2, 21.6±0.2, 33.1±0.2 degrees in a powder X-ray diffraction pattern.

15. A crystal of a compound represented by the formula (IV): 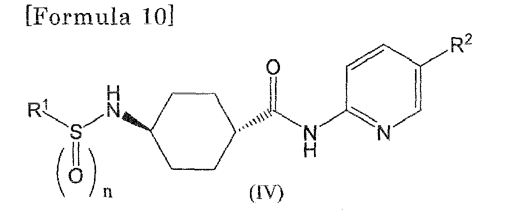 wherein R¹ is tert-butyl, R² is trifluoromethyl, and n is 2, having an endothermic peak at 219.6±2.0°C in a differential scanning calorimetry.

16. A compound represented by the formula (II): 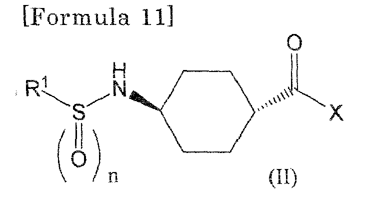 wherein R¹ is substituted or unsubstituted alkyl, n is 2, and X is halogen, its salt or a solvate thereof.
